# DEMANDE DE BREVET EUROPEEN

(11) **EP 0 834 295 A1**
(43) Date de publication de la demande: **08.04.1998**
(21) Numéro de dépôt: 97402279.0
(22) Date de dépôt: 30.09.1997
(51) Int. Cl.: A61F 2/44

(54) **Prothèse d'arthrodèse intersomatique vertébrale**

(30) Priorité: 03.10.1996 FR 9612078
(71) Demandeur: MEDINOV-AMP, 42300 Roanne (FR); Antoine, Jacques Stéphane, 78400 Chatou (FR); Kerboul, Bernard, 29000 Quimper (FR); Lucet, Alain, 21121 Fontaine les Dijon (FR); Pere, Christian, 37260 Artannes sur Indre (FR); Tardivon, Alain, 36000 Chateauroux (FR)
(72) Inventeur: Antoine, Jacques Stéphane, 78400 Chatou (FR); Gauneau, Bertrand, 42300 Roanne (FR); Kerboul, Bernard, 29000 Quimper (FR); Lucet, Alain, 21121 Fontaine les Dijon (FR); Pere, Christian, 37260 Artannes sur Indre (FR); Tardivon, Alain, 36000 Chateauroux (FR)
(74) Mandataire: Martin, Jean-Paul

(57) **Abrégé**

La prothèse d'arthrodèse intersomatique vertébrale, sous forme de cage (1) présente une forme générale de parallélépipède allongé et est destinée à être insérée entre des faces opposées de vertèbres adjacentes d'une colonne vertébrale suivant une direction parallèle à un plan sagittal. Cette cage (1) comporte deux évidements (3, 5) centraux ouverts sur des côtés opposés (9, 10) regardant lesdites faces opposées de vertèbres adjacentes de façon à pouvoir recevoir un greffon osseux et permettre la fusion osseuse avec le tissu des vertèbres adjacentes, et des moyens de blocage anti-retour (25) de ladite cage (1) entre les vertèbres.

Lesdits côtés opposés (9, 10), qui forment respectivement une face supérieure et une face inférieure, comportent chacun un bombement (11, 12), au moins sur une partie, le long d'une dimension longitudinale de la cage. De plus, lesdits côtés opposés (9, 10) s'étendent transversalement à la cage (1) dans des directions générales convergentes.

## Description

La présente invention est relative aux prothèses d'arthrodèse intersomatique vertébrale.

Ces prothèses sont destinées à compenser les effets dûs à un traumatisme ou une dégénération du disque intervertébral qui conduit à un tassement ou une réduction de l'espace discal intersomatique entraînant des répercussions telles que, notamment, la compression des racines nerveuses avec des conséquences connues.

En d'autres termes, le but de ces prothèses est alors de rétablir un espace discal convenable et, dans l'impossibilité, généralement, de rétablir une mobilité articulaire sur prothèse, de réaliser une fusion osseuse des deux corps vertébraux.

On connaît déjà un certain nombre de ces prothèses, dont certaines sont commercialisées, qui se présentent sous forme de fiches destinées à être insérées ou vissées entre deux corps vertébraux, ces fiches étant, généralement, associées par paires et le plus généralement mises en place par voie postérieure.

Le document EP-A-0 307 241 décrit un implant de prothèse chirurgical, souvent appelé cage intersomatique. Ce brevet prévoit de pratiquer, dans les deux plateaux vertébraux en regard et à travers l'espace discal, un canal de section rectangulaire constante dans lequel est insérée la cage intersomatique de forme correspondante contenant un greffon osseux.

La cage intersomatique connue présente une forme sensiblement parallélépipédique et comporte une face antérieure chanfreinée afin de faciliter l'insertion dans le canal de section rectangulaire préalablement pratiqué. Sur ses faces extérieures, cette cage comporte des rugosités ou des aspérités superficielles afin d'empêcher l'éjection de la prothèse. Cette cage comprend en outre des fentes ou des gorges destinées à recevoir un greffon osseux.

Une telle cage est généralement implantée à l'aide d'un outil ancillaire de pose tel qu'un impacteur. A cet effet, la cage intersomatique et l'impacteur comprennent des moyens complémentaires de fixation temporaire.

Cette prothèse connue présente l'inconvénient qu'il faut préparer préalablement un canal de réception de la prothèse. Cette opération augmente considérablement la durée de l'intervention chirurgicale et donc les risques pour les patients.

Un autre type de prothèse est connu de la demande de brevet FR 95 14 655 appartenant à la Demanderesse. Cette demande décrit un implant osseux de forme cylindrique bombée et présentant un filetage périphérique extérieur autotaraudant.

Bien que des canaux de réception de la prothèse préalablement préparés ne soient plus nécessaires, la pose de cette prothèse demande une certaine dextérité, et la durée de l'intervention chirurgicale n'est pas encore optimale.

L'invention vise à pallier les inconvénients susmentionnés en proposant une prothèse d'arthrodèse intersomatique vertébrale qui soit relativement facile à poser, et qui permet donc de réduire la durée de l'intervention chirurgicale. De plus, elle vise à proposer une prothèse qui ne nécessite pas de pratiquer préalablement un canal de réception de la prothèse dans l'espace intervertébral.

L'invention a donc pour objet une prothèse d'arthrodèse intersomatique vertébrale, sous forme de cage présentant une forme générale de parallélépipède allongé et destinée à être insérée entre des faces opposées de vertèbres adjacentes d'une colonne vertébrale suivant une direction parallèle à un plan sagittal, ladite cage comportant au moins un évidement central ouvert sur des côtés opposés regardant lesdites faces opposées de vertèbres adjacentes de façon à pouvoir recevoir un greffon osseux et permettre la fusion osseuse avec le tissu des vertèbres adjacentes, et des moyens de blocage anti-retour de ladite cage entre les vertèbres, caractérisée en ce que lesdits côtés opposés, qui forment respectivement une face supérieure et une face inférieure, comportent chacun un bombement, au moins sur une partie, le long d'une dimension longitudinale de la cage et s'étendent transversalement à la cage, dans des directions générales convergentes.

La prothèse suivant l'invention peut comporter une ou plusieurs des caractéristiques suivantes :
- les bombements le long de la dimension longitudinale de la cage présentent chacun un rayon de courbure compris entre 60mm et 80mm et, notamment de l'ordre de 70mm,
- les bombements le long de la dimension longitudinale de la cage sont sous-tendus par des plans géométriques associés qui font un angle suivant la dimension longitudinale de la cage, de préférence compris entre 4° et 7°, s'ouvrant dans le sens d'insertion de la cage,
- chacun desdits côtés opposés comporte en outre un bombement le long de la dimension transversale de la cage,
- les bombements le long de la dimension transversale de la cage présentent des rayons de courbure compris entre 60mm et 80mm, de préférence de l'ordre de 70mm,
- les directions générales convergentes, suivant lesquelles s'étendent lesdits côtés opposés, font un angle compris entre 5° et 10°,
- la cage comporte une face antérieure dont la largeur est inférieure à sa hauteur, de préférence de 1,5mm,
- les moyens de blocage anti-retour comprennent des nervures transversales de blocage disposées sur lesdits côtés opposés de la cage,
- la cage comporte sur chacun desdits côtés opposés au moins une nervure centrale de blocage disposée à l'endroit le plus bombé de la cage et une nervure distale disposée à l'extrémité antérieure de la cage,
- la cage comporte deux évidements centraux, de préférence de la même taille, séparés l'un de l'autre par une paroi transversale de renforcement,
- la cage comprend une face latérale, notamment la face latérale extérieure, comportant un marquage, notamment un marquage par laser, en vue de l'identification des faces latérales extérieure et intérieure,
- la cage est réalisée en un métal biocompatible, notamment du titane et de l'inox,
- la cage est réalisée en un matériau biocompatible et radiotransparent, notamment du polyétherétherkétone,
- la cage présente un plan central de symétrie orienté perpendiculairement par rapport à un axe défini par l'évidement.

L'invention a également pour objet une paire de prothèses d'arthrodèse intersomatique vertébrale, caractérisée en ce qu'elle comprend deux prothèses telles que définies ci-dessus, destinées à être insérées côte à côte entre les mêmes faces opposées de vertèbres adjacentes d'une colonne vertébrale.

Selon une autre caractéristique, la paire de prothèses définie ci-dessus comprend deux prothèses d'arthrodèse intersomatique vertébrale identiques.

L'invention a encore pour objet un jeu de prothèses d'arthrodèse intersomatique vertébrale caractérisé en ce qu'il comprend une série de paires de prothèses telles que définies ci-dessus et dont la hauteur générale varie d'intervalles progressifs, l'intervalle étant compris entre 1,5 mm et 2,5mm, de préférence égal à 2mm.

D'autres caractéristiques et avantages de l'invention ressortiront de la description suivante, donnée à titre d'exemple, sans caractère limitatif, en regard des dessins annexés sur lesquels :
La figure 1 est une vue en perspective d'une prothèse suivant l'invention;
La figure 2 est une vue en perspective de la prothèse de la figure 1 tournée de 180° autour d'un axe vertical,
La figure 3 est une vue en coupe longitudinale suivant la ligne III/III de la figure 1;
La figure 4 est une vue en coupe transversale suivant la ligne lV/lV de la figure 3;
La figure 5 est une vue frontale de la face antérieure de la prothèse de la figure 1;
La figure 6 est une vue analogue à celle de la figure 4 montrant une variante d'une prothèse suivant l'invention; et
La figure 7 est une vue frontale de deux prothèses d'arthrodèse intersomatique vertébrale selon l'invention insérées côte à côte entre deux vertèbres adjacentes d'une colonne vertébrale.

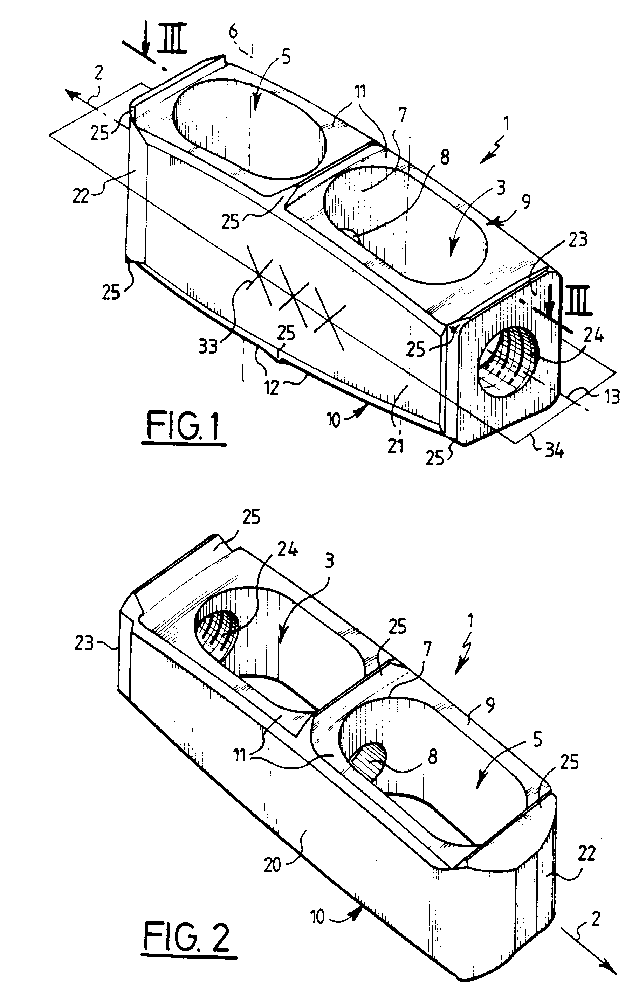

Sur la figure 1 est représentée une prothèse d'arthrodèse intersomatique vertébrale. Cette prothèse se présente sous la forme d'une cage 1 ayant une forme générale de parallélépipède allongé. Cette cage 1 est destinée à être insérée entre les faces discales de deux vertèbres adjacentes d'une colonne vertébrale suivant le sens d'insertion de la cage 1 indiqué par la flèche 2.

La cage 1 comporte deux évidements centraux 3 et 5 de la même taille, destinés à recevoir chacun un greffon osseux, pour permettre la fusion osseuse avec le tissu de vertèbres adjacentes d'une colonne vertébrale. Les évidements 3 et 5 définissent chacun un axe central 6 perpendiculaire à la direction d'insertion 2 de la cage. Ces évidements 3 et 5 sont séparés l'un de l'autre par une paroi transversale de renforcement 7. Cette paroi 7 comporte un trou central 8 de communication pour permettre un contact et une fusion entre les greffons placés dans les évidements 3 et 5.

Comme on le voit sur la figure 2, chaque évidement 3, 5 est ouvert sur des côtés opposés 9 et 10 de la cage 1. Ces côtés 9 et 10 forment respectivement une face supérieure et une face inférieure de la cage 1, et sont en regard, à l'état implanté de la cage, des faces opposées de vertèbres adjacentes.

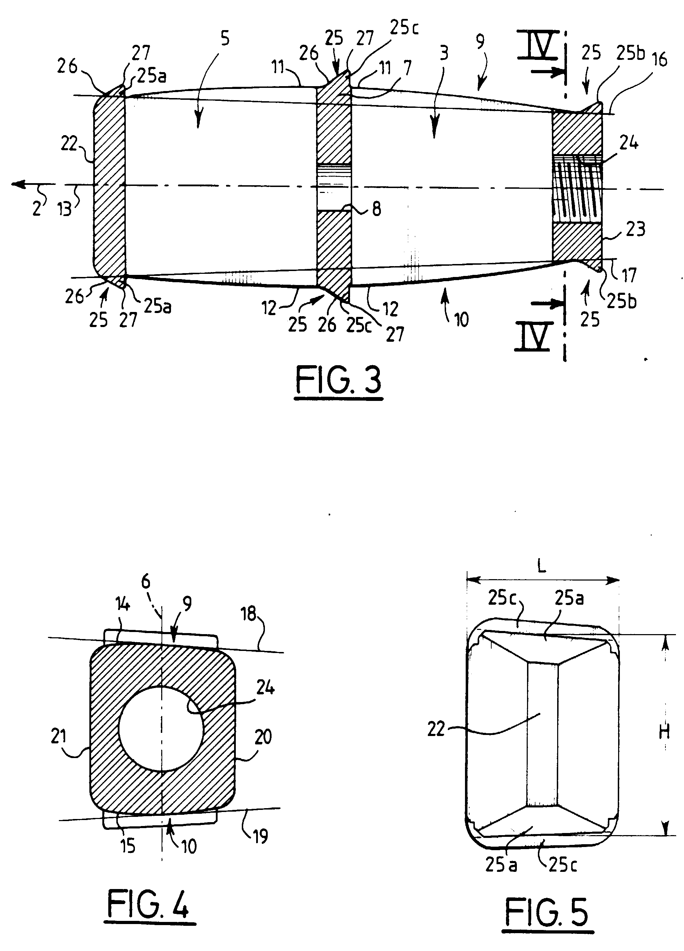

Ainsi que cela est représenté sur les figures 1 et 2, et plus particulièrement sur la figure 3, les côtés 9 et 10 comportent respectivement un bombement 11, 12 le long d'un axe longitudinal 13 de la cage 1. On comprend alors que les côtés 9 et 10 sont bombés sur toute la longueur de la cage 1. Les sommets de courbure des bombements 11 et 12 se trouvent sensiblement au niveau de la paroi 7 de renforcement. Ces bombements 11 et 12 présentent chacun un rayon de courbure compris entre 60mm et 80mm, de préférence de l'ordre de 70mm.

Ainsi qu'on le voit sur la figure 4, chaque côté 9, 10 comporte en outre un bombement 14, 15 le long de la dimension transversale de la cage 1. Les rayons de courbure de chaque bombement 14, 15 sont également compris entre 60mm et 80mm, mais de préférence de l'ordre de 70mm.

En référence à la figure 3, on voit que chaque bombement longitudinal 11, 12 est sous-tendu par un plan géométrique 16, 17 associé. Suivant la dimension longitudinale de la cage 1, ces plans 16 et 17 font un angle, de préférence compris entre 4° et 7°, s'ouvrant dans le sens d'insertion 2 de la cage.

La figure 4 montre que les côtés 9 et 10 s'étendent transversalement à la cage 1 dans des directions générales convergentes. Les directions générales sont définies par des plans associés 18 et 19 tangents respectivement aux bombements transversaux 14 et 15 au niveau de l'axe central 6. Ces plans 18 et 19 associés font transversalement à la cage 1 un angle compris entre 5° et 10°. Cet angle de convergence des plans 18 et 19 est choisi en fonction de la taille de la cage 1.

Grâce d'une part aux bombements longitudinaux 11 et 12 et transversaux 14 et 15 des côtés 9 et 10 et, d'autre part, aux convergences des plans géométriques 16 et 17 ainsi que 18 et 19, les faces supérieure et inférieure de la cage 1 épousent au mieux les faces discales des vertèbres présentant une forme sensiblement concave. En d'autre terme, les faces inférieure et supérieure de la cage 1 présentent une forme sensiblemenmt complémentaire aux faces discales des vertèbres.

Du fait de la convergence des plans 18 et 19, la hauteur de l'une des faces latérales, la face latérale intérieure 20, est plus importante que celle de l'autre face latérale, la face latérale extérieure 21. Bien entendu, les termes "intérieure" et extérieure" sont utilisés selon la signification médicale.

Pour faciliter l'insertion, la cage 1 comporte une face antérieure 22 en biseau (voir figure 2), et une face postérieure 23 destinées à coopérer avec un outil de pose non représenté, tel qu'un impacteur (voir figure 1).

Pour l'impaction, la face postérieure 23 comporte un trou taraudé 24 destiné à coopérer avec un filetage extérieur de l'impacteur non représenté.

Comme on le voit sur les figures 1, 2 et 3, chaque côté 9 et 10 comporte des moyens de blocage anti-retour 25 réalisés sous la forme de nervures transervales de blocage. Ces nervures 25 de blocage servent à empécher une expulsion de la cage 1 lors de et après la pose. Plus en détail, chaque côté 9 et 10 comporte respectivement une nervure distale 25a et une nervure proximale 25b au niveau des extrémités respectivement antérieure et postérieure de la cage 1 ainsi qu'une nervure centrale 25c au niveau de la paroi 7 de renforcement. Chaque nervure 25a, 25b, 25c comporte une rampe 26 d'écartement des vertèbres dont l'inclinaison est opposée au sens d'insertion 2 de la cage afin de faciliter l'introduction de celle-ci dans l'espace intervertébral, et qui se termine en une arête de retenue 27.

Par ailleurs, la figure 5 montre que la largeur L de la face antérieure 22 est inférieure à sa hauteur H, de préférence de 1,5mm.

Afin de pouvoir distinguer facilement les faces latérales 20 et 21 de la cage 1 lors de l'intervention chirurgicale, une des faces, de préférence la face latérale extérieure 21, comporte un marquage 33, notamment un marquage par laser (voir figure 1).

Comme cela est représenté sur les figures 1 et 3, la cage 1 présente un plan central de symétrie 34. Ce plan 34 de symétrie est perpendiculaire par rapport à l'axe 6 défini par un des évidements 3 et 5. Cet axe 6 est à la fois parallèle aux faces latérales 21 et 22 et à la face postérieure 23. Grâce à cette symétrie de la cage 1, on peut implanter côte à côte des paires de cages identiques.

La cage 1 est réalisée en un métal biocompatible tel que le titane ou de l'inox. On prévoit également de réaliser la cage 1 en un matériau biocompatible et radio-transparent, notamment du polyétherétherkétone.

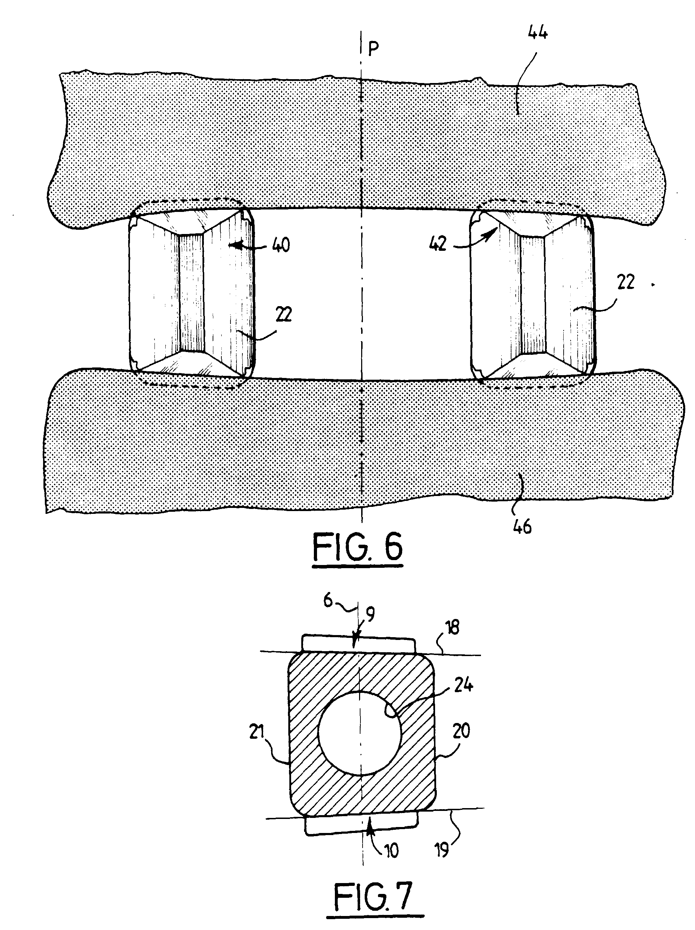

La figure 6 montre une coupe transversale d'une variante de la prothèse suivant l'invention. Sur cette figure, les éléments identiques ont été désignés par les mêmes numéros de référence. Cette variante se distingue de celle représentée sur les figures 1 à 5 essentiellement par le fait que les côtés 9 et 10 de la cage 1 sont inclinés l'un par rapport à l'autre, mais ne comportent pas de bombement transversal. Les plans 18 et 19 définissant les directions générales convergentes se confondent alors avec les côtés 9 et 10 sur la largeur de la cage 1.

La figure 7 montre une paire de cages 40 et 42 identiques à celle des figures 1 à 5, insérées côte à côte de part et d'autre du plan sagittal central P, entre des faces opposées de vertèbres adjacentes 44 et 46 d'une colonne vertébrale. Le fait de pouvoir utiliser une paire de prothèses identiques permet de réduire considérablement le coût de fabrication.

Pour la pose de la prothèse, le chirurgien, après avoir pratiqué un accès, de préférence postérieur, et, dans ce cas, écarté latéralement le tronçon correspondant du névraxe, enlève des débris de disque lombaire. Ensuite, il présente la cage, vissée sur un impacteur, par son extrémité antérieure dans l'espace intervertébral, puis commence à impacter la cage dans un plan sagittal à travers l'espace intervertébral. Grâce à la face antérieure 22 en biseau, et aux bombements 11 et 12 longitudinaux des côtés 9 et 10, les plateaux vertébraux sont écartés progressivement. Lors de cette insertion de la cage par coups, l'expulsion de la cage est empêchée par les nervures 25 qui s'ancrent sur les corticales des plateaux vertébraux se faisant face. Une fois la cage mise en place, sa forme bombée, au niveau de ses parties inférieures respectivement supérieures, s'adapte sensiblement aux formes franchement concave du plateau de la vertèbre supérieure et légèrement concave du plateau de la vertèbre inférieure, ce qui facilite la répartition des efforts et assure une bonne stabilité. On obtient également un meilleur développement du tissu osseux et une bonne coopération avec des greffons osseux préalablement introduits dans les évidements 3, 5 de la cage, conduisant à une bonne arthrodèse. Par ailleurs, un retrait accidentel de la cage est considérablement réduit, du fait que le renflement central de la cage s'oppose à son extraction.

Pour satisfaire aux exigences des interventions chirurgicales, on prévoit un jeu de prothèses comprenant une série de paires de cages d'arthrodèse intersomatique vertébrale dont la hauteur générale varie d'intervalles progressifs. Cette hauteur générale de la cage est définie par la distance entre les arêtes de retenue 27 des nervures centrales 25c disposées respectivement sur les faces supérieure 9 et inférieure 10 de la cage. Avantageusement, l'intervalle est compris entre 1,5 et 2,5mm et de préférence égale à 2mm.

## Revendications

1. Prothèse d'arthrodèse intersomatique vertébrale, sous forme de cage (1) présentant une forme générale de parallélépipède allongé et destinée à être insérée entre des faces opposées de vertèbres adjacentes (44, 46) d'une colonne vertébrale suivant une direction parallèle à un plan sagittal, ladite cage (1) comportant au moins un évidement (3; 5) central ouvert sur des côtés opposés (9, 10) regardant lesdites faces opposées de vertèbres adjacentes (44, 46) de façon à pouvoir recevoir un greffon osseux et permettre la fusion osseuse avec le tissu des vertèbres adjacentes (44, 46), et des moyens de blocage anti-retour (25) de ladite cage (1; 40, 42) entre les vertèbres (44, 46), caractérisée en ce que lesdits côtés opposés (9, 10), qui forment respectivement une face supérieure et une face inférieure, comportent chacun un bombement, au moins sur une partie, le long d'une dimension longitudinale (13) de la cage et s'étendent, transversalement à la cage (1) dans des directions générales convergentes.

2. Prothèse selon la revendication 1, caractérisée en ce que les bombements le long de la dimension longitudinale de la cage présentent chacun un rayon de courbure compris entre 60mm et 80mm et, notamment de l'ordre de 70mm.

3. Prothèse suivant la revendication 1 ou 2, caractérisée en ce que les bombements le long de la dimension longitudinale de la cage sont sous-tendus par des plans (16, 17) géométriques associés qui font un angle suivant la dimension longitudinale de la cage, de préférence compris entre 4° et 7°, s'ouvrant dans le sens d'insertion (2) de la cage.

4. Prothèse selon l'une quelconque des revendications 1 à 3, caractérisée en ce que chacun desdits côtés opposés (9, 10) comporte en outre un bombement le long de la dimension transversale de la cage (1).

5. Prothèse selon la revendication 4, caractérisée en ce que les bombements le long de la dimension transversale de la cage (1) présentent des rayons de courbure compris entre 60mm et 80mm, de préférence de l'ordre de 70mm.

6. Prothèse selon l'une quelconque des revendications 1 à 5, caractérisée en ce que les directions générales convergentes, suivant lesquelles s'étendent lesdits côtés opposés (9, 10), font un angle compris entre 5° et 10°.

7. Prothèse selon l'une quelconque des revendications 1 à 6, caractérisée en ce que la cage (1) comporte une face antérieure (22) dont la largeur (L) est inférieure à sa hauteur (H), de préférence de 1,5mm.

8. Prothèse selon l'une quelconque des revendications 1 à 7, caractérisée en ce que les moyens de blocage anti-retour comprennent des nervures (25) transversales de blocage disposées sur lesdits côtés opposés (9, 10) inférieure et supérieure.

9. Prothèse selon la revendication 8, caractérisée en ce que la cage comporte sur chacun desdits côtés opposés (9, 10) au moins une nervure centrale (25c) de blocage disposée à l'endroit le plus bombé de la cage (1) et une nervure distale (25a) disposée à l'extrémité antérieure de la cage (1).

10. Prothèse selon l'une quelconque des revendications 1 à 9, caractérisée en ce que la cage (1) comporte deux évidements (3, 5) centraux, de préférence de la même taille, séparés l'un de l'autre par une paroi (7) transversale de renforcement.

11. Prothèse selon l'une quelconque des revendications 1 à 10, caractérisée en ce que la cage (1) comprend une face latérale, notamment la face latérale extérieure (21), comportant un marquage (33), notamment un marquage par laser en vue de l'identification des faces latérales extérieure (21) et intérieure (20).

12. Prothèse selon l'une quelconque des revendications 1 à 11, caractérisée en ce que la cage (1) est réalisée en un métal biocompatible, notamment du titane et de l'inox.

13. Prothèse selon l'une quelconque des revendications 1 à 11, caractérisée en ce que la cage (1) est réalisée en un matériau biocompatible et radiotransparent, notamment du polyétherétherkétone.

14. Prothèse selon l'une quelconque des revendications 1 à 13, caractérisée en ce que la cage (1) présente un plan (34) central de symétrie orienté perpendiculairement par rapport à un axe (6) défini par l'évidement (3; 5).

15. Paire de prothèses d'arthrodèse intersomatique vertébrale caractérisée en ce qu'elle comprend deux prothèses (40; 42) suivant l'une quelconque des revendications 1 à 14, destinées à être insérées côte à côte entre les mêmes faces opposées de vertèbres adjacentes (44, 46) d'une colonne vertébrale.

16. Paire de prothèses selon les revendications 14 et 15 prises ensembles, caractérisée en ce qu'elle comprend deux prothèses (40, 42) identiques.

17. Jeu de prothèses d'arthrodèse intersomatique vertébrale caractérisé en ce qu'il comprend une série de paires (40, 42) de prothèses selon la revendication 15 ou 16, dont la hauteur générale varie d'intervalles progressifs, l'intervalle étant compris entre 1,5mm et 2,5mm, de préférence égal à 2mm.
